# EUROPÄISCHE PATENTSCHRIFT

(11) **EP 0 721 934 B1**
(45) Veröffentlichungstag und Bekanntmachung des Hinweises auf die Patenterteilung: **26.08.1998**
(21) Anmeldenummer: 96100056.9
(22) Anmeldetag: 04.01.1996
(51) Int. Cl.: C07C 209/60

(54) **Verfahren zur Herstellung von tert.-Butylamin**
Process for the preparation of tert. butylamine
Procédé pour la fabrication de tert. butylamine

(30) Priorität: 13.01.1995 DE 19500839
(43) Veröffentlichungstag der Anmeldung: 17.07.1996
(73) Patentinhaber: BASF Aktiengesellschaft, 67063 Ludwigshafen (DE)
(72) Erfinder: Dingerdissen, Uwe, Dr., D-64342 Seeheim-Jugenheim (DE); Henne, Andreas, Dr., D-67433 Neustadt (DE); Herrmann, Jürgen, D-68307 Mannheim (DE); Pfeffinger, Joachim, D-67063 Ludwigshafen (DE); Stops, Peter, D-67122 Altrip (DE)

(56) Entgegenhaltungen:
- EP-A- 0 132 736
- EP-A- 0 431 451
- EP-A- 0 560 157
- EP-A- 0 587 424
- WO-A-93/17995

## Beschreibung

Die Erfindung betrifft ein Verfahren zur Herstellung von tert.-Butylamin durch Umsetzung von Isobuten mit Ammoniak an aciden Heterogenkatalysatoren unter konstanter oder nahezu konstanter Temperatur.

Aus der DE-A-33 26 579 ist ein Verfahren zur Herstellung von tert.-Butylamin durch Umsetzung von Isobuten mit Ammoniak an zeolithischen Katalysatoren bekannt. Bei diesem Verfahren werden Ammoniak und Isobuten im Molverhältnis von 1:1 bis zu 5:1 einem Festbettreaktor zugeführt, in dem bei Drücken von 40 bis 700 bar und Temperaturen von 200 bis 350°C die Umsetzung am Katalysator zu tert.-Butylamin stattfindet. Diese Umsetzung ist exotherm und stellt eine Gleichgewichtsreaktion dar. Nachteilig an diesem Verfahren ist, daß unter technischen Reaktionsbedingungen Isobutenumsätze von weniger als 20 % zu erreichen sind. Dadurch müssen die nichtumgesetzten Einsatzstoffe nach dem Reaktor abgetrennt und in den Reaktor zurückgeführt werden, wodurch hohe Betriebskosten entstehen.

Aus Catalysis of Organic Reactions, Marcel Dekker Inc., New York and Basel, Seite 241 bis 260 ist bekannt, daß der thermodynamisch erreichbare Gleichgewichtsumsatz mit zunehmender Temperatur stark abnimmt und daß zur Erzielung von technisch sinnvollen Umsatzraten eine Temperatur von mindestens 250°C nötig ist. Führt man die Reaktion in einem Festbettreaktor unter adiabatischen Betriebsbedingungen durch, so erwärmt sich das Reaktionsgemisch durch die exotherme Umsetzung und der erreichbare Gleichgewichtsumsatz sinkt dadurch stark ab.

Der vorliegenden Erfindung lag daher die Aufgabe zugrunde, den zuvor genannten Nachteilen abzuhelfen.

Demgemäß wurde ein neues und verbessertes Verfahren zur Herstellung von tert.-Butylamin durch Umsetzung von Isobuten mit Ammoniak bei Temperaturen von 200 bis 350°C und Drücken von 40 bis 700 bar in Gegenwart eines aciden Heterogenkatalysators gefunden, welches dadurch gekennzeichnet ist, daß man die Reaktion in einer oder mehreren Reaktionszonen unter jeweils konstanter oder nahezu konstanter Temperatur durchführt.

Das erfindungsgemäße Verfahren kann wie folgt durchgeführt werden:

Die Reaktion kann in einem Rohrbündelreaktor durchgeführt werden, in dem der acide Heterogenkatalysator in den Rohren angeordnet ist und die Rohre von einem Wärmeträgermedium umströmt werden, wodurch die Reaktionsmischung auf einer konstanten oder nahezu konstanten Temperatur, d.h. Temperaturschwankungen von 0 bis 5°C, bevorzugt 0 bis 3°C, besonders bevorzugt 0 bis 2°C, gehalten wird.

Der Rohrbündelreaktor kann so betrieben werden, daß die Reaktionsmischung aus Isobuten und Ammoniak in Strömungsrichtung auf konstanter oder nahezu konstanter Temperatur gehalten wird (isothermer Betrieb). Das aus dem Reaktor erhaltene Produktgemisch kann in einer Destillationskolonne aufgetrennt und das nichtumgesetzte Isobuten und Ammoniak vom tert.-Butylamin abgetrennt und in die Reaktion zurückgeführt werden.

Es ist außerdem möglich, den Rohrbündelreaktor konstruktiv so zu gestalten, daß der Außenraum um die Rohre in mehrere räumlich voneinander getrennte Einzelräume aufgetrennt wird und jeder dieser Einzelräume mit einem eigenen Wärmeträgerstrom beaufschlagt wird. Diese Heizzonen können dabei so betrieben werden, daß das Reaktionsmedium in Strömungsrichtung auf konstanter Temperatur gehalten wird oder bevorzugt so betrieben werden, daß die Temperatur der Reaktionsmischung in Strömungsrichtung absinkt.

Weiterhin ist es möglich, die Reaktion in mehreren, hintereinander angeordneten Rohrbündelreaktoren durchzuführen, wobei die Reaktoren unabhängig voneinander von einem Wärmeträgermedium auf konstanter oder nahezu konstanter Temperatur gehalten werden können oder bevorzugt die Temperatur des ersten Reaktors höher, z.B. 2 bis 20°C, bevorzugt 3 bis 10°C höher, liegt als die Temperaturen der nachfolgenden Reaktoren.

Eine ebenfalls günstige Verfahrensdurchführung kann durch Verwendung eines Festbettreaktors mit eingebauten Rohren erzielt werden, wobei der acide Heterogenkatalysator um die Rohre herum angeordnet wird und die Rohre von einem Wärmeträgermedium durchströmt werden, wodurch es möglich ist, die Reaktionsmischung auf einer konstanten oder nahezu konstanten Temperatur zu halten.

Eine weitere mögliche Ausführungsform besteht darin, daß ein Festbettreaktor verwendet wird, in welchen in Strömungsrichtung an einer oder an mehreren Stellen kaltes Isobuten und/oder kaltes Ammoniak, jeweils von Temperaturen von 0 bis 50°C, bevorzugt 10 bis 35°C, besonders bevorzugt Raumtemperatur (20 bis 25°C) zugeführt wird, wodurch die Reaktionsmischung abkühlt und dadurch ein höherer Gleichgewichtsumsatz möglich wird.

Dem mit Katalysator gefüllten Festbettreaktor kann an mehreren Stellen Isobuten und/oder Ammoniak zugeführt werden, wobei die Temperaturen dieser zugeführten Ströme kleiner sind als die Reaktoreintrittstemperatur [in der Regel 0 bis 50°C, bevorzugt 10 bis 35°C, besonders bevorzugt Raumtemperatur (20 bis 25°C)].

Bei Verwendung eines Festbettreaktors ist es ebenso möglich, an einer oder an mehreren Stellen des Reaktors ein Teil der Reaktionsmischung oder die gesamte Reaktionsmischung zu entnehmen, durch einen Wärmeaustauscher zu leiten, abzukühlen und wieder dem Reaktor zuzuführen, so daß die Temperatur der Reaktionsmischung nach dem Wärmeaustauscher 240 bis 280°C, bevorzugt 250 bis 270°C beträgt.

Dabei ist der Reaktor in der Regel so aufgebaut, daß der Katalysator in mehreren Einzelfestbetten (Horden) angeordnet ist, wobei die Reaktionsmischung nach jeden Einzelfestbett aus dem Reaktor herausgeführt wird, mittels eines Wärmeaustauschers abgekühlt und wiederum dem Reaktor zugeführt wird. Die Wärmeaustauscher können konstruktiv auch innerhalb des Reaktors angeordnet werden.

Das erfindungsgemäße Verfahren kann auch in zwei oder in mehreren, also 2 bis 10, bevorzugt 2 bis 5, besonders bevorzugt 2 bis 4 räumlich voneinander getrennten Festbettreaktoren durchgeführt werden, wobei die Reaktionsmischung zwischen den einzelnen Apparaten durch Wärmeaustauscher oder durch Zuführung von kaltem Isobuten und/oder Ammoniak abgekühlt wird.

Bei allen diesen Ausführungformen des Reaktors ist es möglich, den gleichen oder unterschiedliche acide Heterogenkatalysatoren zu verwenden.

Als acide Heterogenkatalysatoren eignen sich acide oberflächenreiche Silicate, insbesondere Alumo- und Borosilikate. Hierbei kann es sich um röntgenamorphe oder kristalline Verbindungen handeln. Bevorzugt ist die Verwendung von Zeolithen. Insbesondere die Verwendung von Pentasilen, B-Pentasilen, Ferrierite, ZSM-48, ZSM-23, ZSM-22, Fanjasit, BETA, Mordenit, Offretit, Omega, L-Zeolith, ZSM-12.

Die Zeolithe können unterschiedliche chemische Zusammensetzung aufweisen, z.B. Alumino-, Eisen-, Gallium-, Chrom-, Arsen- und Wismutsilikatzeolithe oder deren Gemische, sowie Alumino-, Gallium-und Eisengermanatzeolithe oder deren Gemische.

Insbesondere eignen sich die Aluminosilikat- oder Eisensilikatzeolithe. Geeignete Aluminosilikat- oder Eisensilikatzeolithe erhält man z.B. aus einer Aluminiumverbindung, vorzugsweise Al(OH)3 oder Al₂(SO₄)₃ bzw. Eisenverbindung, vorzugsweise Fe₂(SO₄)₃, und einer Siliciumverbindung, vorzugsweise hochdisperses Siliciumdioxid, in wäßriger Aminlösung, insbesondere in 1,6-Hexamethylendiamin-oder 1,3-Propandiamin- oder Triethylentetramin-Lösung mit und ohne Alkali- oder Erdalkalizusatz bei 100 bis 22O°C unter autogenem Druck. Die synthetisierten Aluminosilikatzeolithe haben je nach Wahl der Einsatzstoffmengen ein SiO₂/Al₂O₃-Verhältnis von 10 bis 40 000.

Geeignete Aluminosilikat- und Eisensilikatzeolithe kann man auch in etherischem Medium wie Diethylenglykoldimethylether, in alkoholischem Medium wie Methanol bzw. 1,4-Butandiol aber auch in Wasser synthetisieren.

Die so hergestellten Aluminosilikat- und Eisensilikatzeolithe kann man nach ihrer Isolierung, Trocknung bei 100 bis 160°C, vorzugsweise bei etwa 110°C, und Calcinierung bei 450 bis 550°C, vorzugsweise bei etwa 500°C, mit einem Bindemittel zu Strängen oder Tabletten verformen. Als Bindemittel eignen sich verschiedene Aluminiumoxide bevorzugt Boehmith, amorphe Aluminosilikate Siliciumdioxid, bevorzugt hochdisperses SiO₂ sowie Ton. Nach der Verformung werden die Extrudate oder Preßlinge zweckmäßig bei 110°C/16 h getrocknet und bei 500°C/16 h calciniert.

Eine besondere Ausführungsform besteht darin, daß der isolierte Aluminosilikat- oder Eisensilikatzeolith direkt nach der Trocknung verformt wird und erstmals nach der Verformung einer Calcination unterworfen wird.

Zur Erhöhung der Selektivität, der Standzeit und der Anzahl der möglichen Regenerierungen kann man verschiedene Modifizierungen an den zeolithischen Katalysatoren vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie Mn, Fe, Mo, Cu, Zn, Pd, Pt, Ag und Seltenen Erdmetallen wie La, Ce ionenaustauschen bzw. dotieren kann.

Eine vorteilhafte Ausführungsform besteht darin, daß man die verformten Pentasilzeolithe in einem Strömungsrohr vorlegt und bei 20 bis 100°C, z.B. ein Halogenid oder ein Nitrat der oben beschriebenen Metalle überleitet. Ein derartiger Ionenaustausch kann z.B. an der Wasserstoff-, Ammonium- und Alkaliform der Zeolithe vorgenommen werden.

Eine weitere Möglichkeit der Metallaufbringung auf die Zeolithe besteht darin, daß man das zeolithische Material, z.B. mit einem Halogenid, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen kann eine Nachbehandlung mit Wasserstoff und/oder mit Wasserdampf günstig sein.

Eine weitere Möglichkeit der Modifizierung besteht darin, daß man das zeolithische Material - verformt oder unverformt - einer Behandlung mit Säuren wie Salzsäure, Flußsäuren und Phosphorsäure unterwirft.

Weitere Formen der Modifizierung sind der DE-A-33 26 579 zu entnehmen.

Tert.-Butylamin ist ein wichtiges Zwischenprodukt in der Kautschuk- und Gummiindustrie und wird zur Herstellung von Industriechemikalien, Pflanzenschutzmitteln und Farbstoffen verwendet.

### Beispiele

### Beispiel 1

Für eine kontinuierliche Herstellung von tertiär-Butylamin wurde ein Hochdruckreaktor mit 300 mm Länge (Füllhöhe des Katalysators) und einem Innendurchmesser von 50 mm eingesetzt, der ohne Zufuhr oder Abfuhr von Wärme betrieben wurde (adiabate Fahrweise). Der Reaktor wurde mit 300 g (580 ml) Katalysator mit einem mittleren Partikeldurchmesser von 1,5 mm gefüllt. Als Katalysator wurde ein mit 1,5 Gew.-Anteilen Chrom dotierter Borzeolith (Modul: 34) eingesetzt. Ammoniak und Isobuten wurden im molaren Verhältnis von 1,5:1 gemischt, erhitzt und dem Reaktor in einer Menge von 1750 g/h (entspricht 4 g/h Isobuten pro 1 g Katalysator) bei einem Druck von 300 bar zugeführt. Die Eintrittstemperatur betrug 270°C, die Austrittstemperatur betrug 295°C.

Der Reaktoraustrag wurde entspannt und gaschromatographisch analysiert.

Der Umsatz des Isobutens betrug 12,8 % bei einer Selektivität zu tert.-Butylamin von >99,5%.

### Beispiel 2

Für eine kontinuierliche Herstellung wurde ein Hochdruckreaktor mit 500 mm Länge (Füllhöhe des Katalysators) und 25 mm Innendurchmesser eingesetzt, welcher mittels Öltemperierung auf konstanter Temperatur gehalten wurde (isothermer Betrieb). Der Reaktor wurde mit 130 g (250 ml) Katalysator gefüllt. Die Art des Katalysators und der mittlere Partikeldurchmesser ist wie unter Beispiel 1 beschrieben. Die Reaktoreintrittstemperatur sowie die Austrittstemperatur betrug 290°C. Die Ergebnisse sind in Tabelle 1 zusammengestellt.

### Beispiel 3

Für eine kontinuierliche Herstellung wurde ein Hochdruckreaktor wie in Beispiel 1 verwendet, wobei die Temperatur des Reaktors so geregelt wurde, daß längs des Reaktors in Strömungsrichtung eine kontinuierliche Temperabsenkung erreicht wurde. Die Reaktoreintrittstemperatur betrug 300°C, die Austrittstemperatur betrug 280°C. Die Ergebnisse sind in Tabelle 1 zusammengestellt.

### Beispiel 4

Für eine kontinuierliche Herstellung wurden zwei hintereinander angeordnete, adiabtisch betriebene Hochdruckreaktoren mit 100 mm Länge (Reaktor 1), 50 mm Innendurchmesser und 200 mm Länge (Reaktor 2), 50 mm Innendurchmesser verwendet. Zwischen den beiden Reaktoren wurde ein Wärmeaustauscher angeordnet, welcher die Reaktionsmischung abkühlt. Im 1. Reaktor befanden sich 100 g Katalysator, im 2. Reaktor 200 g Katalysators der gleichen Zusammensetzung und Partikelgröße wie in Beispiel 1 beschrieben. Die Reaktoreintrittstemperatur des 1. Reaktors betrug 280°C, die Eintrittstemperatur des 2. Reaktors ebenfalls 280°C; die Austrittstemperatur des 2. Reaktors 290°C. Die Ergebnisse sind in Tabelle 1 zusammengestellt.

### Beispiel 5

Für eine kontinuierliche Herstellung wurden zwei hintereinander angeordnete, adiabtisch betriebene Hochdruckreaktoren mit 100 mm Länge (Reaktor 1), 50 mm Innendurchmesser und 200 mm Länge (Reaktor 2), 50 mm Innendurchmesser verwendet. Zwischen den beiden Reaktoren wurde kalte Isobuten/Ammoniak-Mischung zur Abkühlung der Reaktionsmischung zudosiert. Die Gesamtmenge an Isobuten/Ammoniak-Einsatzmischung wurde dabei so aufgeteilt, daß 2/3 der Menge dem 1. Reaktor zugeführt wurde, die restliche Menge vor dem 2. Reaktor zugefügt wurde. Im 1. Reaktor befanden sich 100 g Katalysator, im 2. Reaktor 200 g Katalysators der gleichen Zusammensetzung und Partikelgröße wie in Beispiel 1 beschrieben. Die Reaktoreintrittstemperatur des 1. Reaktors betrug 280°C, die Eintrittstemperatur des 2. Reaktors 275°C; die Austrittstemperatur des 2. Reaktors 290°C. Die Ergebnisse sind in Tabelle 1 zusammengestellt.

Bei allen durchgeführten Versuchen lag die Selektivität der Reaktion zu tertiär-Butylamin > 99,5 %. Der verwendete Katalysator zeigte während der Versuche keinen Aktivitätsverlust.

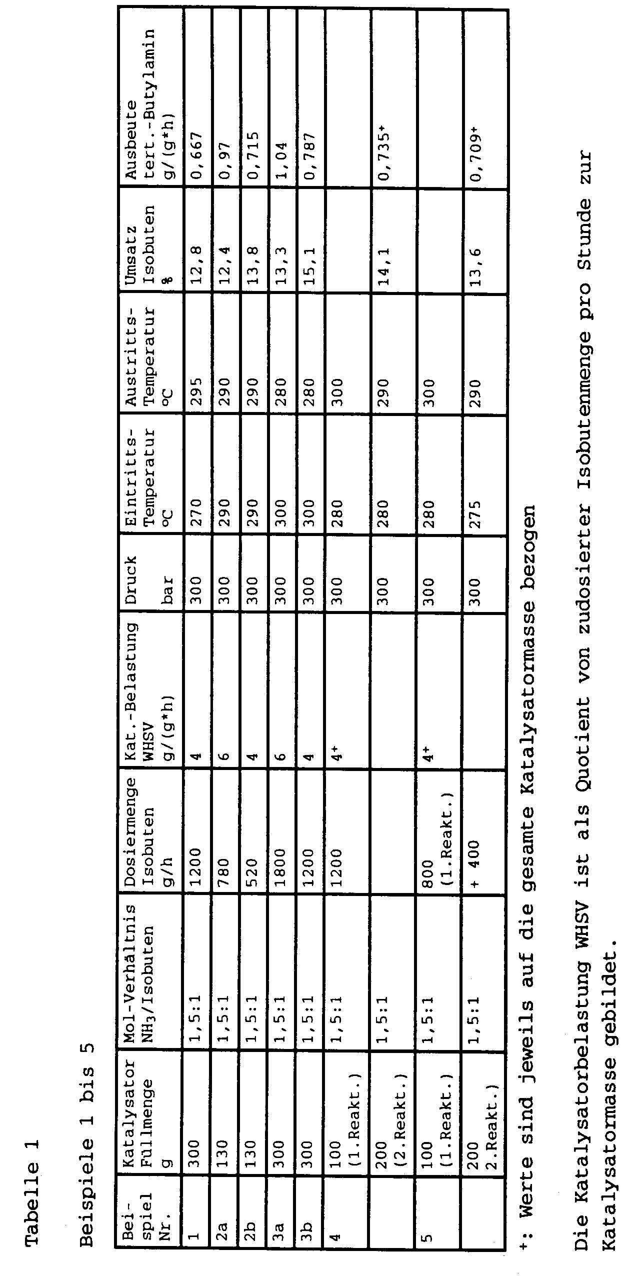

## Patentansprüche

1. Verfahren zur Herstellung von tert. -Butylamin durch Umsetzung von Isobuten mit Ammoniak bei Temperaturen von 200 bis 350°C und Drücken von 40 bis 700 bar in Gegenwart eines aciden Heterogenkatalysators, dadurch gekennzeichnet, daß man die Reaktion in einer oder mehreren Reaktionszonen unter jeweils konstanter oder nahezu konstanter Temperatur durchführt.

2. Verfahren zur Herstellung von tert.-Butylamin nach Anspruch 1, dadurch gekennzeichnet, daß man die Reaktion in einem Rohrbündelreaktor durchführt.

3. Verfahren zur Herstellung von tert.-Butylamin nach Anspruch 1, dadurch gekennzeichnet, daß man einen Rohrbündelreaktor einsetzt, der in Strömungsrichtung mindestens zwei voneinander getrennte Reaktionszonen aufweist, die jeweils durch ein Wärmeträgermedium auf konstanter oder nahezu konstanter Temperatur gehalten werden und die Temperatur der ersten Reaktionszone um 2 bis 20°C höher gehalten wird als die Temperatur der nachfolgenden Reaktionszonen.

4. Verfahren zur Herstellung von tert.-Butylamin nach Anspruch 1, dadurch gekennzeichnet, daß man die Reaktion in einem Festbettreaktor durchführt.

5. Verfahren zur Herstellung von tert.-Butylamin nach Anspruch 1, dadurch gekennzeichnet, daß man die Reaktion in einem Festbettreaktor durchführt und in Strömungsrichtung an einer oder an mehreren Stellen kaltes Isobuten und/oder Ammoniak zur Abkühlung der Reaktionsmischung zuführt.

6. Verfahren zur Herstellung von tert.-Butylamin nach Anspruch 1, dadurch gekennzeichnet, daß man die Reaktion in einem Festbettreaktor durchführt, in dem man die Reaktionsmischung in Strömungsrichtung an einer oder mehreren Stellen dem Reaktor entnimmt, durch einen Wärmeaustauscher führt und abkühlt und wieder dem Reaktor zuführt.

7. Verfahren zur Herstellung von tert.-Butylamin nach Anspruch 1, dadurch gekennzeichnet, daß man die Reaktion in mindestens zwei aufeinanderfolgenden Festbettreaktoren durchführt und die Reaktionsmischung zwischen den einzelnen Festbettreaktoren durch Zuführung von kalten Isobuten und/oder Ammoniak abkühlt.

8. Verfahren zur Herstellung von tert.-Butylamin nach Anspruch 1, dadurch gekennzeichnet, daß man die Reaktion in mindestens zwei aufeinanderfolgenden Festbettreaktoren durchführt und die Reaktionsmischung zwischen den einzelnen Festbettreaktoren durch Wärmeaustauscher abkühlt.

9. Verfahren zur Herstellung von tert.-Butylamin nach Anspruch 1, dadurch gekennzeichnet, daß man in der ersten und den folgenden Reaktionszonen den gleichen Heterogenkatalysator einsetzt.

10. Verfahren zur Herstellung von tert.-Butylamin nach Anspruch 1, dadurch gekennzeichnet, daß man in der ersten und den folgenden Reaktionszonen unterschiedliche Heterogenkatalysatoren einsetzt.

## Claims

1. A process for preparing tert-butylamine by reacting isobutene with ammonia in the presence of an acidic heterogeneous catalyst at from 200 to 350°C and from 40 to 700 bar, which comprises carrying out the reaction in one or more reaction zones, in each case at constant or virtually constant temperature.

2. A process for preparing tert-butylamine as claimed in claim 1, wherein the reaction is carried out in a tube bundle reactor.

3. A process for preparing tert-butylamine as claimed in claim 1, wherein a tube bundle reactor is used which has, in the direction of flow, at least two separate reaction zones which are maintained in each case at virtually constant or constant temperature by a heat transfer medium and the first reaction zone is kept at a temperature which is from 2 to 20°C higher than the temperature of the subsequent reaction zones.

4. A process for preparing tert-butylamine as claimed in claim 1, wherein the reaction is carried out in a fixed-bed reactor.

5. A process for preparing tert-butylamine as claimed in claim 1, wherein the reaction is carried out in a fixed-bed reactor and cold isobutene and/or ammonia is/are added in the direction of flow at one or more locations to cool the reaction mixture.

6. A process for preparing tert-butylamine as claimed in claim 1, wherein the reaction is carried out in a fixed-bed reactor in which the reaction mixture is discharged from the reactor in the direction of flow at one or more locations, passed through a heat exchanger and cooled down and recycled into the reactor.

7. A process for preparing tert-butylamine as claimed in claim 1, wherein the reaction is carried out in at least two consecutive fixed-bed reactors and the reaction mixture is cooled down between the individual fixed-bed reactors by addition of cold isobutene and/or ammonia.

8. A process for preparing tert-butylamine as claimed in claim 1, wherein the reaction is carried out in at least two consecutive fixed-bed reactors and the reaction mixture is cooled down between the individual fixed-bed reactors by means of heat exchangers.

9. A process for preparing tert-butylamine as claimed in claim 1, wherein the same heterogeneous catalyst is used in the first and subsequent reaction zones.

10. A process for preparing tert-butylamine as claimed in claim 1, wherein different heterogeneous catalysts are used in the first and subsequent reaction zones.

## Revendications

1. Procédé de préparation de tert.-butylamine par réaction d'isobutène avec de l'ammoniac à des températures de 200-350°C et sous des pressions de 40-700 bar, en présence d'un catalyseur hétérogène acide, caractérisé en ce que l'on mène la réaction dans une ou plusieurs zones réactionnelles à des températures à chaque fois constantes ou quasi-constantes.

2. Procédé de préparation de tert.-butylamine selon la revendication 1, caractérisé en ce que l'on mène la réaction dans un réacteur à faisceaux de tubes.

3. Procédé de préparation de tert.-butylamine selon la revendication 1, caractérisé en ce que l'on utilise un réacteur à faisceaux de tubes présentant, dans le sens du flux, au moins deux zones réactionnelles séparées qui sont chacune maintenues à température constante ou quasi-constante au moyen d'un agent caloporteur, la première zone réactionnelle étant maintenue à une température supérieure de 2-20°C à celle de la zone réactionnelle suivante.

4. Procédé de préparation de tert.-butylamine selon la revendication 1, caractérisé en ce que l'on mène la réaction dans un réacteur à lit fixe.

5. Procédé de préparation de tert.-butylamine selon la revendication 1, caractérisé en ce que l'on mène la réaction dans un réacteur à lit fixe et en ce que l'on introduit, dans le sens du flux, à un ou plusieurs endroits, de l'isobutène froid et/ou de l'ammoniac pour refroidir le mélange réactionnel.

6. Procédé de préparation de tert.-butylamine selon la revendication 1, caractérisé en ce que l'on mène la réaction dans un réacteur à lit fixe, et en ce que l'on prélève du mélange réactionnel, dans le sens du flux, à un ou plusieurs endroits du réacteur, on le conduit à travers un échangeur de chaleur et on le refroidit, puis on l'introduit à nouveau dans le réacteur.

7. Procédé de préparation de tert.-butylamine selon la revendication 1, caractérisé en ce que l'on mène la réaction dans au moins deux réacteurs à lit fixe en série et en ce que l'on refroidit le mélange réactionnel entre les deux réacteurs à lit fixe par introduction d'isobutène froid et/ou d'ammoniac.

8. Procédé de préparation de tert.-butylamine selon la revendication 1, caractérisé en ce que l'on mène la réaction dans au moins deux réacteurs à lit fixe en série et en ce que l'on refroidit le mélange réactionnel entre les deux réacteurs à lit fixe au moyen d'un échangeur de chaleur.

9. Procédé de préparation de tert.-butylamine selon la revendication 1, caractérisé en ce que l'on introduit le même catalyseur hétérogène dans la première zone réactionnelle et dans celle qui la suit.

10. Procédé de préparation de tert.-butylamine selon la revendication 1, caractérisé en ce que l'on introduit des catalyseurs hétérogènes différents dans la première zone réactionnelle et dans celle qui la suit.
